# EUROPEAN PATENT APPLICATION

(11) **EP 4 799 627 A1**
(43) Date of publication of application: **02.09.2026**
(21) Application number: 26160898.8
(22) Date of filing: 26.02.2026
(51) Int. Cl.: A61K 31/7048, A61K 36/21, A61K 36/42, A61K 36/752, A61P 9/14, A23L 33/105, A61P 7/00

(54) **MIXTURE, COMPOSITION COMPRISING IT, AND THEIR USE IN THE PREVENTIVE AND/OR CURATIVE TREATMENT OF VENOUS INSUFFICIENCY**

(30) Priority: 27.02.2025 IT 202500003966
(71) Applicant: Ceres Pharma Italy S.r.l., 25124 Brescia (IT)
(72) Inventor: STEFANELLI, Giorgio, 25124 BRESCIA (IT)
(74) Representative: Delbarba, Andrea

(57) **Abstract**

The present invention relates to a mixture comprising diosmin, hesperidin, a pumpkin extract and an amaranth extract, and to a nutraceutical and/or pharmaceutical composition comprising it. In addition, the invention relates to use of the mixture or the composition comprising it in the prevention and/or in the treatment of a venous disorder.

## Description

### FIELD OF THE INVENTION

The present invention relates to a mixture, a nutraceutical and/or pharmaceutical composition comprising it, and their use in the prevention and/or in the treatment of a venous disorder.

### STATE OF THE ART

Venous insufficiency is a pathological condition caused by a difficult return of venous blood to the heart. Over 30% of women in Italy currently suffer from this pathology, although there is currently an increase in cases in men. This incidence could worsen, however, as it is closely linked to the poor dietary habits and sedentary lifestyles typical of Western contexts.

Particularly in cases of functional venous insufficiency, the main risk factors are linked to difficulties in walking, obesity, sedentary lifestyle, unhealthy lifestyle and postural pathologies.

The symptoms in the case of venous insufficiency, particularly in the case of chronic venous insufficiency, are swelling, swollen ankles, oedema of the affected limb, varicose veins, tingling, itchiness, cramps in the calves and also pain.

Diagnosis consists of direct medical observation of the lesions and, in certain cases, it might be necessary to perform a Doppler ultrasound to assess functioning of the veins.

Based on the root cause, venous insufficiency can be classified into two macro-groups: organic venous insufficiency, caused by pathological alterations of the veins, and functional venous insufficiency, a condition caused by a functional overload of the veins which, despite being fully healthy, are forced to work beyond their capabilities.

Stasis dermatitis (inflammation of the skin of the legs, generated by vascular stasis), deep vein thrombosis (a pathological condition due to obstruction of a vein by a blood clot) and the presence of varicose veins (anomalous and permanent dilations of veins and arteries, expression of an alteration of the efficiency of venous valves) are classified as types of organic venous insufficiency.

Lymphoedema and reduced mobility of the limbs, on the other hand, belong to the category of functional venous insufficiency.

Lymphoedema is a clinical condition characterised by lymphatic stasis in the various districts of the organism, caused by an impairment of the lymphatic system. Lymphoedema causes the veins to be overloaded and consequently, in certain cases, venous insufficiency can occur.

Treatment of the various types of venous insufficiency starts with correction of dietary habits and the adoption of a healthy lifestyle. However, it is often necessary to use elastic compression stockings and, particularly in cases of chronic venous insufficiency, surgery is necessary, in particular valvuloplasty and radiofrequency ablation/laser therapy, in addition to use of anticoagulant and profibrinolytic drugs that cause a considerable number of side effects.

There is therefore a need to have products (mixtures and/or compositions) which can be used in the prevention and/or in the treatment of venous insufficiency.

It is desirable to have products (mixtures and/or compositions) which can be used in the prevention and/or in the treatment of venous insufficiency that avoid the use of invasive treatments, such as valvuloplasty, and that allow to hasten recovery from the complications associated with venous insufficiency, simultaneously guaranteeing a better quality of life and stabilisation of the clinical situation of the patient, and that are more effective than the products currently available on the market, such as, for example, the DFN product comprising diosmin and hesperidin as the active ingredients, and excipients.

Furthermore, it is also necessary to provide products that do not cause side effects and that can therefore be administered to a broad category of people, including immunocompromised individuals.

These aims, and still others, which will emerge clearly from the detailed description that follows, are achieved by the mixtures and the compositions comprising said mixtures (mixture(s) and/or composition(s) of the invention or according to the invention) which have the technical features claimed in the appended claims.

### SUMMARY OF THE INVENTION

Following an intense research activity, the Applicant has developed and prepared a mixture comprising or, alternatively, consisting of specific active components which are all natural ingredients and thus make both the mixture and the pharmaceutical composition or nutraceutical composition or supplement composition comprising such mixture reasonably free of side effects and characterised by high tolerability.

A first aspect of the present invention relates to a mixture comprising or, alternatively, consisting of diosmin, hesperidin, a pumpkin extract and an amaranth extract. Preferably, said diosmin and said hesperidin are present in a diosmin:hesperidin weight ratio comprised from 15:1 to 8:1 by weight.

Preferably, the diosmin is present in a quantity by weight comprised between 20% and 80% and the hesperidin is present in a quantity by weight comprised between 1% and 25% relative to the total weight of the mixture.

In one embodiment, the diosmin is present in a quantity by weight comprised between 30% and 60% and the hesperidin is present in a quantity by weight comprised between 2% and 20% relative to the total weight of the mixture. Preferably, the diosmin is present in a quantity by weight comprised between 40% and 50% and the hesperidin is present in a quantity by weight comprised between 4% and 15% relative to the total weight of the mixture.

The pumpkin extract is preferably obtained from the seeds and/or from the pulp of a plant belonging to the *Cucurbita maxima* species.

Preferably, the amaranth extract is obtained from the seeds of a plant belonging to the *Amaranthus caudatus* and/or *Amaranthus cruentus* and/or *Amaranthus hypochondriacus* species, more preferably, it is obtained from the seeds of a plant belonging to the *Amaranthus caudatus* species.

In one embodiment, the mixture comprises the pumpkin extract in a quantity by weight, relative to the total weight of the mixture, comprised from 20% to 80%, preferably from 30% to 60%, more preferably from 40% to 50%; and the amaranth extract in a quantity by weight, relative to the total weight of the mixture, comprised from 1% to 20%, preferably from 5% to 15%, more preferably from 7% to 10%.

A second aspect of the present invention relates to a composition comprising a mixture described above and, optionally, pharmaceutically acceptable or food-grade additives and/or excipients.

Preferably, the mixture is formulated for oral administration, more preferably as tablets, more preferably, gastro-resistant tablets, extended-release tablets, sublingual tablets, orodispersible tablets, capsules, more preferably hard or soft capsules, coated tablets, oral powders, oral granules, single-dose sachets, oral suspensions, oral solutions, syrups, elixirs, oral drops, oral emulsions, oral pastes, sticks, orosoluble sticks or gels.

A third aspect of the present invention relates to the mixture or the composition described above for use as a medicament.

A fourth aspect of the present invention relates to the mixture or the composition described above for use in the treatment or in the prevention of a venous disorder, preferably a peripheral venous disorder or a pathology caused by or associated with said venous disorder.

Preferably, the venous disorder is chosen from among chronic venous insufficiency, oedema of lower limbs, telangiectasia, varicose veins, reticular veins, capillary fragility, haemorrhoids, skin hyperpigmentation (ochre dermatitis), lipodermatosclerosis, varicose eczema, venous ulcers, deep vein thrombosis (DVT), superficial thrombophlebitis, tissue fibrosis, stasis skin infections, functional venous pain, orthostatic oedema, sensation of warmth or burning along the vein path.

Preferably, the invention relates to use of the mixture or the composition described above for the treatment or prevention of a venous disorder, through an increase in the venous wall tone, improvement of the contractility and elasticity of the venous wall, reduction of tissue inflammation, decrease in vascular permeability, improvement of lymphatic drainage, protection of capillaries.

A fourth aspect of the present invention relates to a non-therapeutic use of the mixture or the composition described above for maintaining or improving microcirculation in an individual.

A fifth aspect of the present invention relates to a dietary supplement comprising the mixture or the composition described above.

### DESCRIPTION OF THE FIGURES

The present invention is also better described with the aid of the following figures, which are provided solely by way of example and, therefore, do not limit the scope thereof.

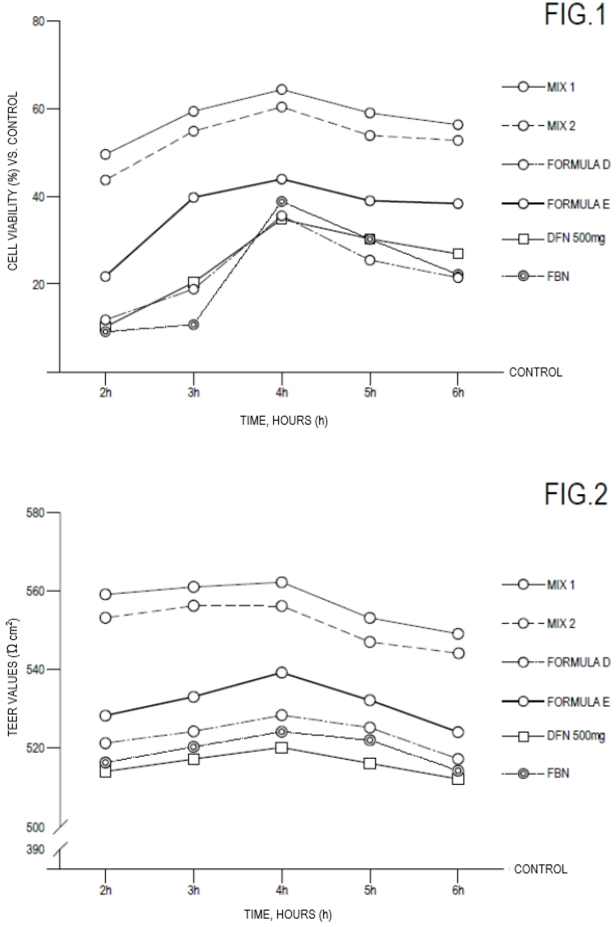

Figure 1 shows the results of toxicity analysis performed on the *in vitro* cell model described in example 1. The data reported are expressed as the mean ± SD of 5 independent experiments conducted in triplicate. All the mixtures are statistically significant on the control (p<0.05).
Figure 2 shows the results of TEER analysis performed on the *in vitro* cell model described in example 1. The data reported are expressed as the mean ± SD of 5 independent experiments conducted in triplicate. All the substances are statistically significant on the control (p<0.05).

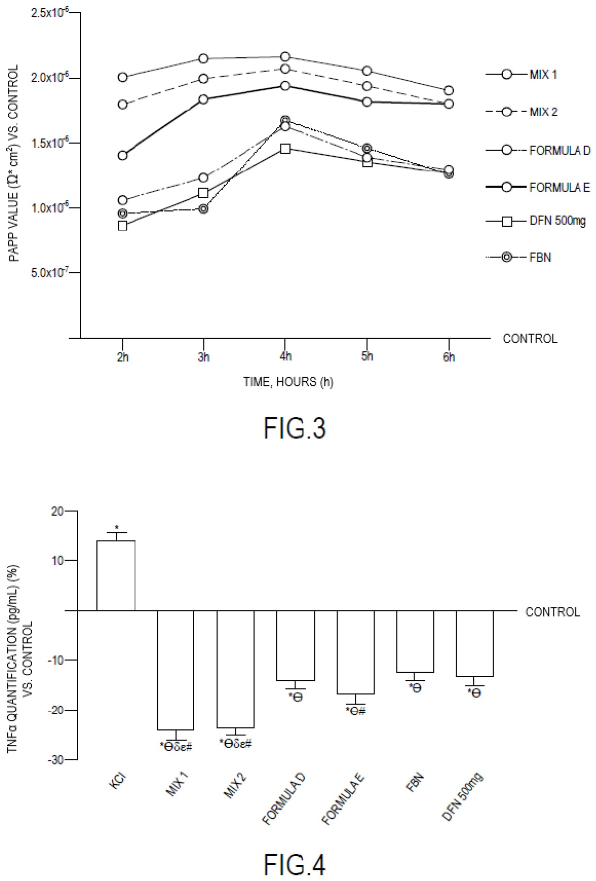

Figure 3 shows the Papp values of the mixtures tested in example 1 and indicated in the figure. The data reported are expressed as the mean ± SD of 5 independent experiments conducted in triplicate. For all the substances, the results obtained between 1h and 6h are statistically significant on the control (p<0.05).
Figure 4 shows the TNFα levels found following treatment of the *in vitro* model described in step 2 of example 1 with the mixtures indicated in the figure. The data reported are expressed as the mean ± SD of 5 independent experiments conducted in triplicate. * p<0.05 vs. control; δ p<0.05 vs. Formula D; ε p<0.05 vs. Formula E; # p<0.05 vs. commercial products; θ p<0.05 vs. KCI.

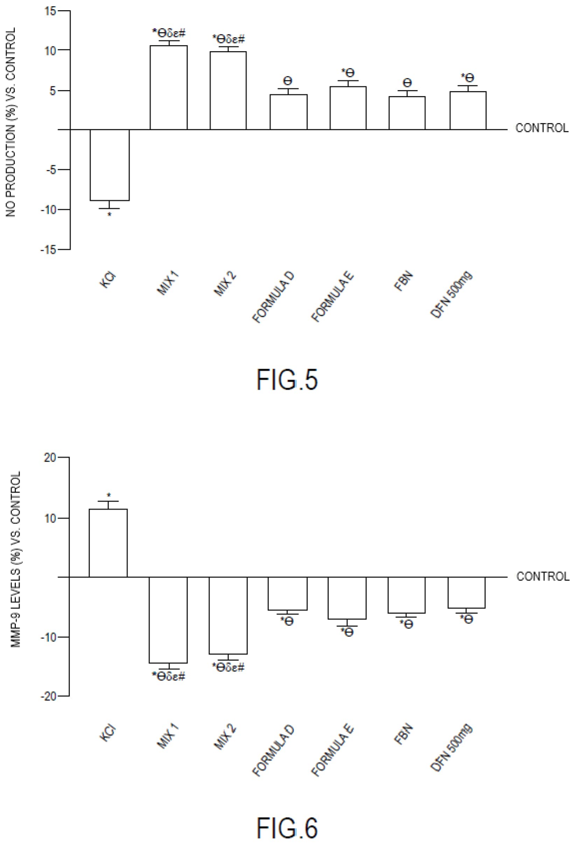

Figure 5 shows the NO production levels induced by treatment of the *in vitro* model described in step 2 of example 1 with the mixtures indicated in the figure. The data reported are expressed as the mean ± SD of 5 independent experiments conducted in triplicate. * p<0.05 vs. control; δ p<0.05 vs. Formula D; ε p<0.05 vs. Formula E; # p<0.05 vs. commercial products; θ p<0.05 vs. KCI.
Figure 6 shows the MMP-9 production levels induced by treatment of the *in vitro* model described in step 2 of example 1 with the mixtures indicated in the figure. The data reported are expressed as the mean ± SD of 5 independent experiments conducted in triplicate. * p<0.05 vs. control; δ p<0.05 vs. Formula D; ε p<0.05 vs. Formula E; # p<0.05 vs. commercial products; θ p<0.05 vs. KCI.

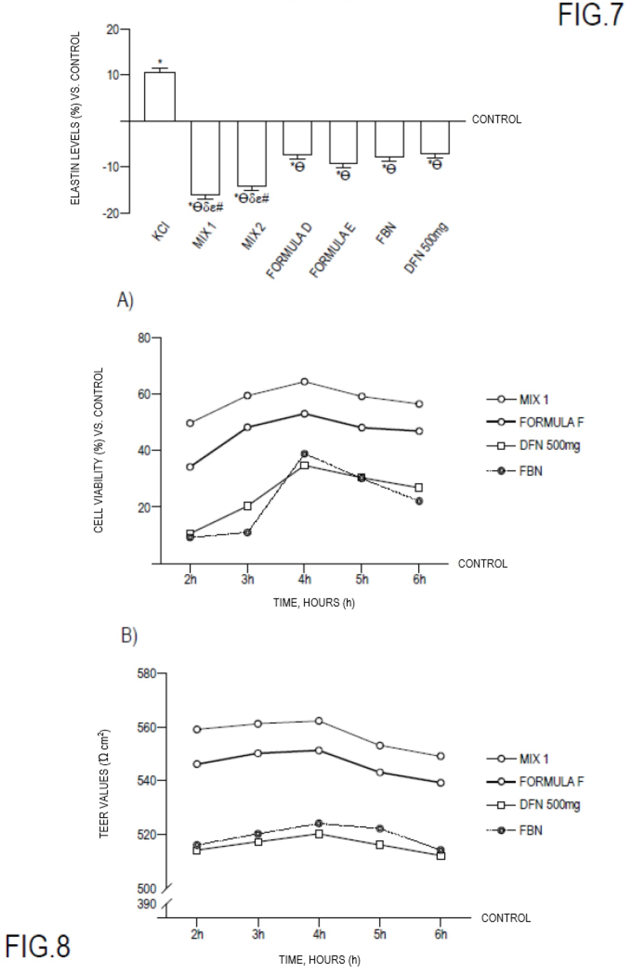

Figure 7 shows the elastin levels induced by treatment of the *in vitro* model described in step 2 of example 1 with the mixtures indicated in the figure. The data reported are expressed as the mean ± SD of 5 independent experiments conducted in triplicate. * p<0.05 vs. control; δ p<0.05 vs. Formula D; ε p<0.05 vs. Formula E; # p<0.05 vs. commercial products; θ p<0.05 vs. KCI.
Figure 8 shows the results of A) toxicity analysis and B) barrier integrity analysis performed on the *in vitro* cell model described in example 2. The data reported are expressed as the mean ± SD of 5 independent experiments conducted in triplicate. All the mixtures are statistically significant on the control (p<0.05).

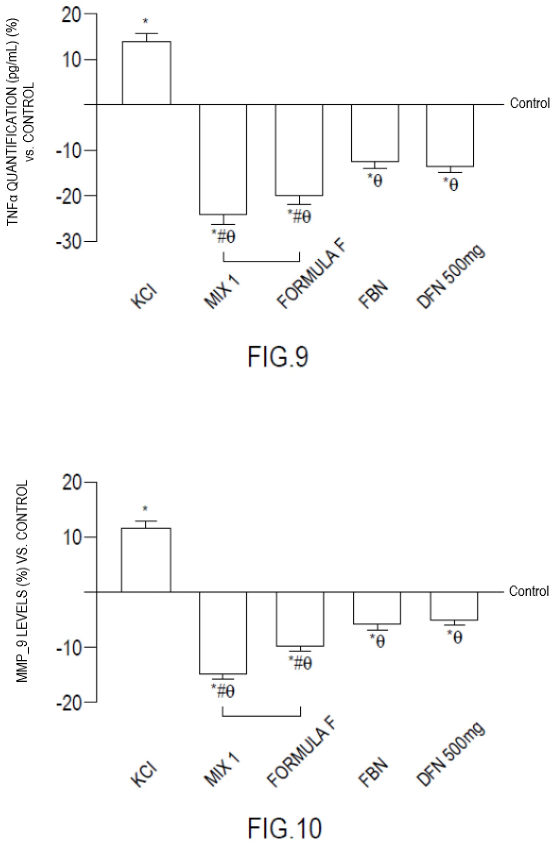

Figure 9 shows the TNFα levels found following treatment of the *in vitro* model of venous insufficiency with the mixtures according to example 2 and indicated in the figure. The data reported are expressed as the mean ± SD of 5 independent experiments conducted in triplicate. * p<0.05 vs. control; # p<0.05 vs. commercial products; θ p<0.05 vs. KCl; bar p<0.05 vs. Formula F.
Figure 10 shows the MMP-9 production levels induced by treatment of the *in vitro* model of venous insufficiency with the mixtures according to example 2 and indicated in the figure. The data reported are expressed as the mean ± SD of 5 independent experiments conducted in triplicate. * p<0.05 vs. control; # p<0.05 vs. commercial products; θ p<0.05 vs. KCl; bar p<0.05 vs. Formula F.

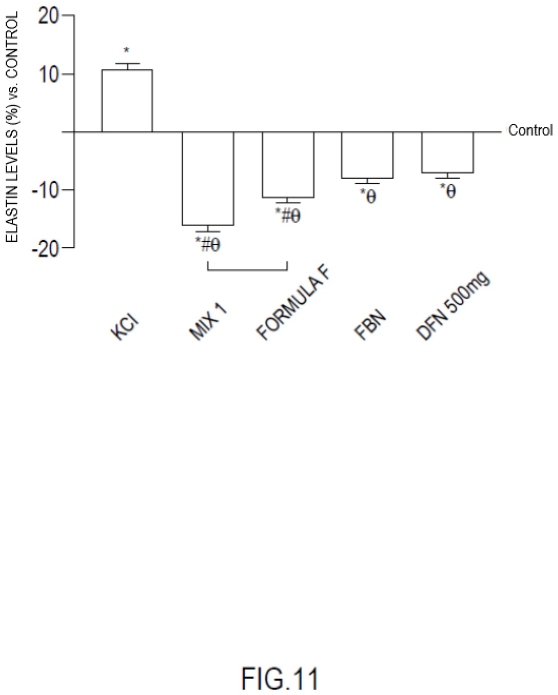

Figure 11 shows the elastin levels induced by treatment of the *in vitro* model of venous insufficiency with the mixtures according to example 2 and indicated in the figure. The data reported are expressed as the mean ± SD of 5 independent experiments conducted in triplicate. * p<0.05 vs. control; # p<0.05 vs. commercial products; θ p<0.05 vs. KCl; bar p<0.05 vs. Formula F.

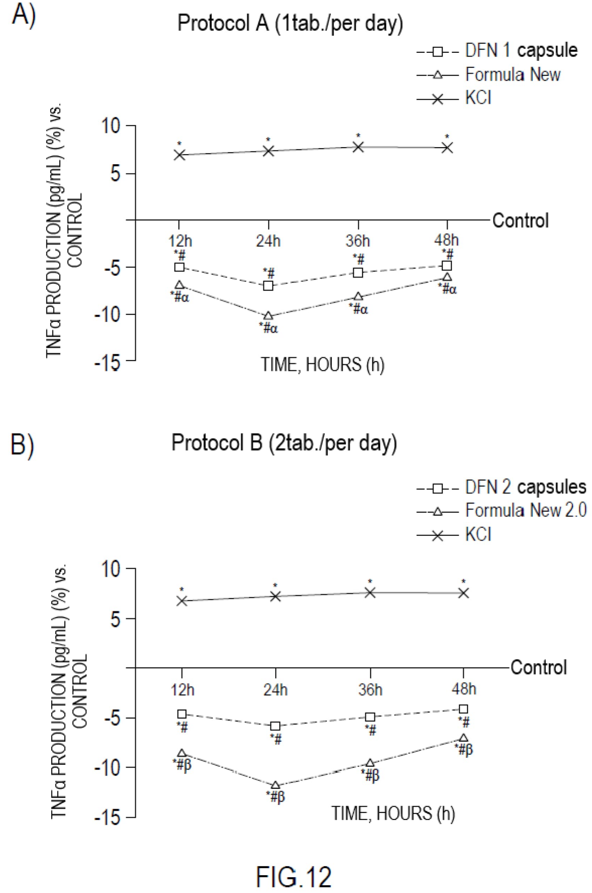

Figure 12 shows the TNFα levels found following treatment of the *in vitro* model of venous insufficiency with the mixtures according to example 3 and indicated in the figure. A) The data reported are expressed as the mean ± SD of 5 independent experiments conducted in triplicate. * p<0.05 vs. control; # p<0.05 vs. KCl; α p<0.05 vs. DFN. B) The data reported are expressed as the mean ± SD of 5 independent experiments conducted in triplicate. * p<0.05 vs. control; # p<0.05 vs. KCl; β p<0.05 vs. DFN.

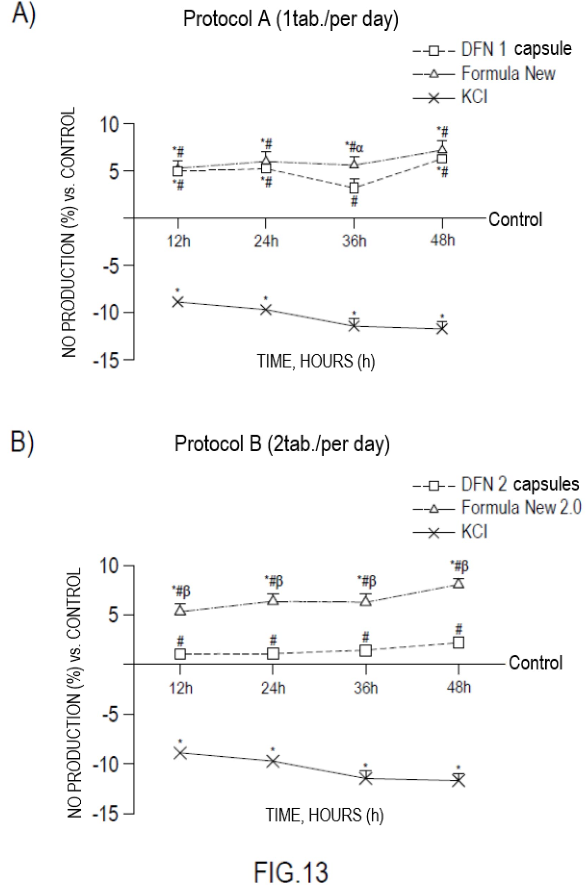

Figure 13 shows the NO production levels induced by treatment of the *in vitro* model of venous insufficiency with the mixtures according to example 3 and indicated in the figure. A) The data reported are expressed as the mean ± SD of 5 independent experiments conducted in triplicate. * p<0.05 vs. control; # p<0.05 vs. KCl; α p<0.05 vs. DFN. B) The data reported are expressed as the mean ± SD of 5 independent experiments conducted in triplicate. * p<0.05 vs. control; # p<0.05 vs. KCl; β p<0.05 vs. DFN.

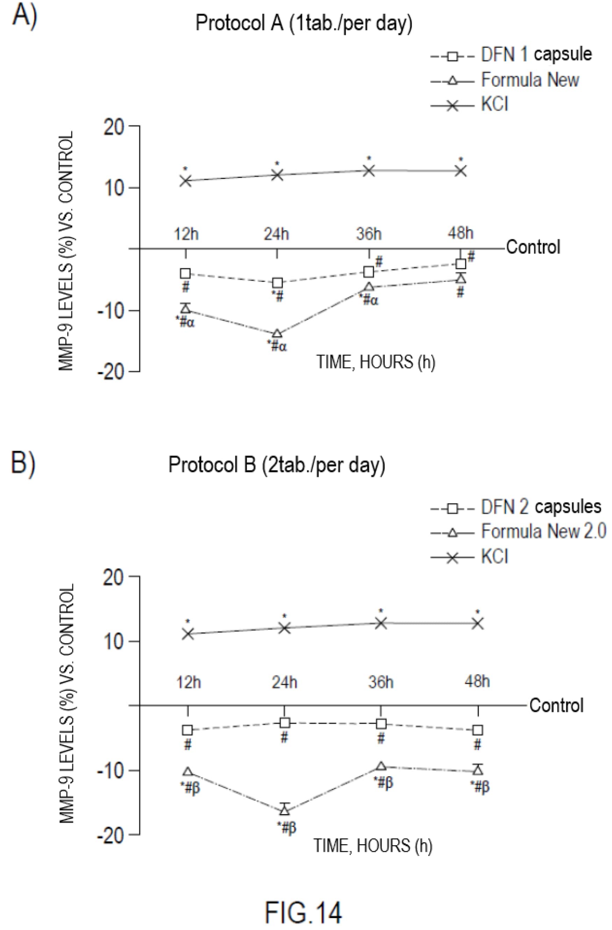

Figure 14 shows the MMP-9 production levels induced by treatment of the *in vitro* model of venous insufficiency with the mixtures according to example 3 and indicated in the figure. A) The data reported are expressed as the mean ± SD of 5 independent experiments conducted in triplicate. * p<0.05 vs. control; # p<0.05 vs. KCl; α p<0.05 vs. DFN. B) The data reported are expressed as the mean ± SD of 5 independent experiments conducted in triplicate. * p<0.05 vs. control; # p<0.05 vs. KCl; β p<0.05 vs. DFN.

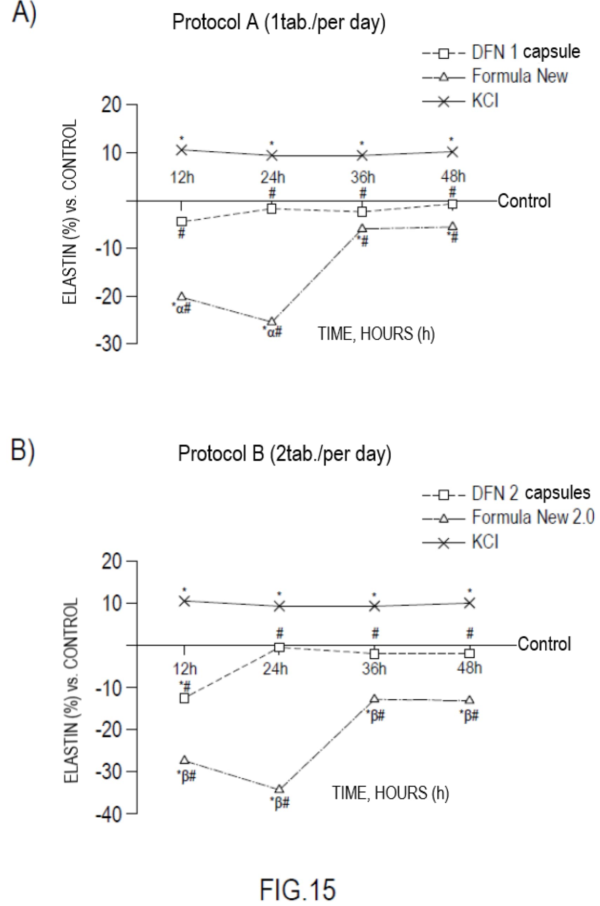

Figure 15 shows the elastin levels induced by treatment of the *in vitro* model of venous insufficiency with the mixtures according to example 2 and indicated in the figure. A) The data reported are expressed as the mean ± SD of 5 independent experiments conducted in triplicate. * p<0.05 vs. control; # p<0.05 vs. KCl; α p<0.05 vs. DFN. B) The data reported are expressed as the mean ± SD of 5 independent experiments conducted in triplicate. * p<0.05 vs. control; # p<0.05 vs. KCl; β p<0.05 vs. DFN.

### DETAILED DESCRIPTION OF THE INVENTION

A **first** aspect of the present invention relates to a mixture comprising diosmin, hesperidin, a pumpkin extract and an amaranth extract.

In one embodiment, the mixture consists of diosmin, hesperidin, a pumpkin extract and an amaranth extract.

Preferably, the diosmin is a semi-synthetic molecule belonging to the flavonoids family, which has molecular formula C₂₈H₃₂O₁₅ and preferably CAS number 520-27-4.

In particular, the diosmin is a semi-synthetic molecule modified starting from the hesperidin molecule and preferably having molecular formula C₂₈H₃₄O₁₅ and CAS number 520-26-3.

The diosmin and/or the hesperidin present in the mixture according to the invention can preferably be molecules of synthetic or semi-synthetic origin.

In the context of the present invention, the term "synthetic origin" indicates a substance that exists in nature and that is formulated by means of a chemical process.

In the context of the present invention, the term "semi-synthetic origin" indicates a natural substance modified in part through chemical synthesis.

In one embodiment, said diosmin and said hesperidin are present in the mixture in a diosmin:hesperidin weight ratio comprised from 15:1 to 8:1.

Preferably, the mixture comprises a phytocomplex comprising diosmin and/or hesperidin, preferably said phytocomplex is an extract of the *Citrus sinensis (L.) Osbeck* fruit.

In one embodiment, the phytocomplex comprises diosmin:hesperidin in a 75:25 weight ratio, preferably 80:20, even more preferably 90:10.

Preferably, the diosmin and the hesperidin have an average particle size for at least 90%, preferably 95%, lower than 10 microns, preferably lower than or equal to 5 microns.

In a further preferred embodiment, the diosmin and the hesperidin are formulated in the form of a microemulsion, in which the diosmin and the hesperidin are incorporated inside a vector, preferably a lipid vector, more preferably micelles.

In several embodiments, the vector comprises linseed oil and hemp seed oil. Preferably, the vector, more preferably a micelle, comprises a linseed oil and hemp seed oil mixture in a volume/volume ratio comprised between 60:40 and 95:5, preferably between 65:35 and 85:15.

The ratio between linseed oil and hemp seed oil is preferably comprised between 70:30 and 85:15, more preferably between 70:30 and 80:20, even more preferably between 60:40 and 95:5, even more preferably between 65:35 e 85:15, and even more preferably around 75:25.

In any case, in the present context, the ratio between linseed oil and hemp seed oil can be modified, inside the range envisaged by the present invention, in particular so as to optimise the efficacy of the composition, for example based on one or more further substances that can be present in the same composition, as described below.

Preferably, the phytocomplex has a minimum diosmin content ≥ 75%, more preferably comprised from 78% to 95%, even more preferably comprised from 85% to 90% measured through HPLC, by weight relative to the total weight of the extract and/or a minimum hesperidin content preferably ≥ 5%, preferably comprised from 8% to 20%, more preferably from 9% to 15% measured through HPLC, by weight relative to the total weight of the extract.

Preferably, the phytocomplex has an iodine content ≤ 0.1%, in sulphated ash ≤ 0.3%, a water content ≤ 6%, in accordance with the standards of the Pharmacopoeia.

Preferably, the mixture according to the invention comprises diosmin in a quantity by weight, relative to the total weight of the mixture, comprised from 20% to 80%, preferably from 30% to 60%, more preferably from 40% to 50%.

Preferably, the mixture according to the invention comprises hesperidin in a quantity by weight, relative to the total weight of the mixture, comprised from 1% to 25%, preferably from 2% to 20%, more preferably from 4% to 15%.

Preferably, said diosmin and said hesperidin are present in the mixture according to the invention in a diosmin:hesperidin weight ratio comprised from 15:1 to 1:15, more preferably in a weight ratio comprised from 10:1 to 1:10, even more preferably in a weight ratio comprised from 5:1 to 1:5; for example in a diosmin:hesperidin weight ratio of 8:1 or 9:1 or 10:1 or 11:1 or 12:1.

Preferably, the pumpkin extract is an extract obtained from the seeds and/or from the pulp of *Cucurbita maxima* (briefly, *C*. *maxima*).

Preferably, said extract from the seeds and/or pulp of *C*. *maxima* is obtained using ethanol as the extraction solvent.

More preferably, said pumpkin extract is a dry extract obtained from the seeds and/or from the pulp of *Cucurbita maxima,* even more preferably said pumpkin extract is a dry extract from the seeds of *Cucurbita maxima.*

Preferably, said pumpkin extract obtained from the seeds and/or from the pulp of *Cucurbita maxima* comprises one or more bioactive components chosen from the group that comprises or, alternatively, consists of: alkaloids, flavonoids, phenols, carbohydrates, tannins, saponins, terpenoids and glycosides.

Preferably, the mixture according to the invention comprises a pumpkin extract obtained from the seeds and/or from the pulp of *Cucurbita maxima,* preferably a dry extract.

Preferably, said pumpkin extract is a dry extract, preferably obtained from the seeds of *Cucurbita maxima Duch. ex Lam.*

In a preferred embodiment of the invention, the pumpkin extract is a composition that comprises a pumpkin extract and at least 10%, preferably 15%, more preferably 20% by weight of maltodextrins. Preferably, the composition comprising pumpkin extract and maltodextrins is in the form of a yellow-brown powder, preferably with a particle size of min. 95% lower than 100 mesh, preferably lower than 80 mesh.

Preferably, the mixture according to the invention comprises the pumpkin extract in a quantity by weight, relative to the total weight of the mixture, preferably comprised from 20% to 80%, preferably from 30% to 60%, more preferably from 40% to 50%.

Preferably, the amaranth extract is present as a dry extract obtained from the seeds of a plant belonging to the *Amaranthus caudatus* and/or *Amaranthus cruentus* and/or *Amaranthus hypochondriacus* species.

More preferably, said amaranth extract is present as a dry extract obtained from the seeds of the *Amaranthus caudatus* plant.

For example, the mixture according to the invention comprises an extract of amaranth seeds having, preferably, CAS number 223747-79-3.

Preferably, the mixture according to the invention comprises an amaranth extract in a quantity by weight, relative to the total weight of the mixture, comprised from 1% to 20%, preferably from 5% to 15%, more preferably from 7% to 10%.

In one embodiment, the amaranth extract is the dry extract of *Amaranthus caudatus L.* having CAS number 223747-79-3.

Said amaranth extract is preferably obtained from the seeds of the *Amaranthus caudatus L* plant through extraction with water.

Preferably, the dry extract of *Amaranthus caudatus L.* comprises at least 90% of particles with an average size lower than 300 microns, preferably lower than 300 microns.

Preferably, said amaranth extract is a lipophilic extract of amaranth seeds comprising one or more of the following bioactive components: tocopherols, natural tocotrienols (vitamin E complex), plant sterols (phytosterols), carotenoids and squalene.

Preferably, said amaranth extract present in the mixture according to the invention comprises palmitic acid in a quantity comprised from 5% to 40%, preferably from 10% to 30%, more preferably from 15% to 25% by weight, relative to the total weight of the extract.

Preferably, said amaranth extract present in the mixture according to the invention comprises stearic acid in a quantity comprised from 0.5% to 15%, preferably from 1% to 10%, more preferably from 2% to 5% by weight, relative to the total weight of the extract.

Preferably, said amaranth extract present in the mixture according to the invention comprises oleic acid in a quantity comprised from 5% to 50%, preferably from 10% to 40%, more preferably from 15% to 35% by weight, relative to the total weight of the extract.

Preferably, said amaranth extract present in the mixture according to the invention comprises oleic acid in a quantity comprised from 20% to 80%, preferably from 30% to 60%, more preferably from 40% to 50% by weight, relative to the total weight of the extract.

Preferably, said amaranth extract comprises squalene in a quantity by weight comprised from 1% to 20%, preferably from 2% to 15%, even more preferably from 5% to 12% by weight, relative to the total weight of the extract.

Preferably, said pumpkin extract and said amaranth extract are present in a pumpkin extract:amaranth extract weight ratio comprised from 10:1 to 01:10, more preferably in a weight ratio comprised from 8:1 to 1:8, even more preferably in a weight ratio comprised from 6:1 to 1:6; for example in a pumpkin extract:amaranth extract weight ratio of 5:1 or 4:1 or 3:1 or 2:1 or 1:1.

Preferably, the mixture according to the invention comprises or, alternatively, consists of:
i) diosmin in a quantity by weight, relative to the total weight of the mixture, preferably comprised from 20% to 80%, preferably from 30% to 60%, more preferably from 40% to 50%; and
ii) hesperidin in a quantity by weight, relative to the total weight of the mixture, preferably comprised from 1% to 25%, preferably from 2% to 20%, more preferably from 4% to 15%; and
iii) pumpkin extract in a quantity by weight, relative to the total weight of the mixture, preferably comprised from 20% to 80%, preferably from 30% to 60%, more preferably from 40% to 50%; and
iv) amaranth extract in a quantity by weight, relative to the total weight of the mixture, preferably comprised from 1% to 20%, preferably from 5% to 15%, more preferably from 9% to 12%.

More preferably, the mixture according to the invention comprises or, alternatively, consists of:
i) diosmin in a quantity by weight, relative to the total weight of the mixture, preferably comprised from 35% to 45% by weight, for example 41% by weight; and
ii) hesperidin in a quantity by weight, relative to the total weight of the mixture, preferably comprised from 4% to 10%, for example 4.5%; and
iii) pumpkin extract in a quantity by weight, relative to the total weight of the mixture, preferably comprised from 42% to 48%, for example 45.5%; and
iv) amaranth extract in a quantity by weight, relative to the total weight of the mixture, preferably comprised from 8% to 10%, for example 9%.

A **second** aspect of the present invention relates to a composition comprising the mixture according to the first aspect of the invention and, optionally, pharmaceutically acceptable or food-grade additives and/or excipients. Preferably, the composition comprises the mixture described above in a quantity by weight comprised from 10% to 90%, preferably comprised from 25% to 75%, more preferably comprised from 40% to 60% relative to the weight of the composition.

Preferably, the composition comprises at least one vehicle and/or excipient in a quantity by weight comprised from 90% to 10%, preferably from 75% to 25%, more preferably comprised from 60% to 40% by weight, relative to the total weight of the composition.

The pharmaceutically acceptable or food-grade additives and/or excipients optionally present in the composition can be chosen from among all the substances known to a person skilled in the pharmaceutical or food preparation art.

The types of food, nutraceutical and/or pharmaceutical additives used to prepare the compositions of the invention, the ratios of the additive contents relative to the active ingredients and the methods for preparing the food, nutraceutical and/or pharmaceutical composition can be appropriately selected by the person skilled in the art.

The composition can also contain sweetening agents, preservative agents and flavouring agents.

In one embodiment, the composition is formulated for oral administration, preferably as tablets, preferably, gastro-resistant tablets, extended-release tablets, sublingual tablets, orodispersible tablets, capsules, preferably hard or soft capsules, coated tablets, oral powders, oral granules, single-dose sachets, oral suspensions, oral solutions, syrups, elixirs, oral drops, oral emulsions, oral pastes, sticks, orosoluble sticks or gels. The tablets may have different shapes among those known in the field of pharmaceutical forms, such as, for example, a cylindrical or spheroidal shape. The tablets may be coated or film-coated with one or more coating or film layers capable of passing through the gastric barrier, according to known methods.

The gel capsules may consist of hard gelatine or soft gelatine or soft gel.

Preferably, the oral composition of the invention is a solid or semi-solid (gel) composition as described above. However, if desired or necessary, the composition may be formulated in liquid form, for example by dissolution or suspension in water.

The composition of the invention, preferably formulated in dosage units, may be administered once or several times a day, for example once or twice a day, preferably twice a day.

The compositions of the invention can be prepared by mixing the single components, and any conventional excipients and/or vehicles.

In particular, both the mixture of the invention and the compositions comprising them are easy to prepare and inexpensive, considering that they can be prepared using the apparatus and preparation processes known to the person skilled in the art and in pharmaceutical practice or in the sector of nutritional supplements.

**A third** aspect of the present invention relates to the mixture or the composition described above for use as a medicament.

A **fourth** aspect of the present invention relates to the composition described above for use in the treatment or in the prevention of a venous disorder, preferably a peripheral venous disorder or a pathology caused by or associated with said venous disorder.

In one embodiment, the venous disorder is chosen from among chronic venous insufficiency, oedema of lower limbs, telangiectasia, varicose veins, reticular veins, capillary fragility, haemorrhoids, skin hyperpigmentation (ochre dermatitis), lipodermatosclerosis, varicose eczema, venous ulcers, deep vein thrombosis (DVT), superficial thrombophlebitis, tissue fibrosis, stasis skin infections, functional venous pain, orthostatic oedema, sensation of warmth or burning along the vein path.

Preferably, the invention relates to the mixture or the composition described above for use in the treatment or prevention of a venous disorder, preferably a peripheral venous disorder or a pathology caused by or associated with said venous disorder, through an increase in the venous wall tone, improvement of the contractility and elasticity of the venous wall, reduction of tissue inflammation, decrease in vascular permeability, improvement of lymphatic drainage, protection of capillaries.

In one embodiment, the mixture or the composition described above are for use in the treatment or in the prevention of a venous disorder in an immunocompromised individual.

A **fifth** aspect of the present invention relates to a non-therapeutic use of the mixture or the composition described above for maintaining or improving microcirculation in an individual. Preferably, the mixture or the composition is administered to an individual not suffering from a venous disorder, preferably not suffering from a peripheral venous disorder.

In one embodiment, the use comprises at least a step of the mixture or the composition described above being administered to or taken by an individual who needs it, preferably to or by an individual not suffering from a venous disorder, preferably not suffering from a peripheral venous disorder.

A **sixth** aspect of the present invention relates to a dietary supplement comprising the mixture and/or the composition described above and optionally at least one excipient and/or at least one vehicle.

### EXAMPLES

### EXAMPLE COMPOSITIONS

### Composition 1

Composition in the form of a tablet or sachet or orosoluble stick or gel stick (1 dosage form twice a day) containing:
225 mg diosmin;
25 mg hesperidin;
250 mg pumpkin (dry ext., seeds);
50 mg amaranth (dry ext., seeds); and,
optionally, pharmaceutically acceptable or food-grade additives and/or excipients. One example, purely illustrative and non-limiting, of a composition that comprises several pharmaceutically acceptable or food-grade additives and/or excipients is shown below in

**Table 1:**

| **Ingredients** | **mg/tab.** | **Daily dose (mg/2tab.)** |
|---|---|---|
| **Microcrystalline cellulose (cellulose gel)** | **406** | **812** |
| **Diosmin and Hesperidin from Citrus /Citrus sinensis (L.) Osbeck fructus)** | **289** | **578** |
| **of which Diosmin** | **225** | **450** |
| **of which Hesperidin** | **25** | **50** |
| **Pumpkin (Cucurbita maxima -Duch, semen) dry extract** | **250** | **500** |
| **Calcium carbonate** | **100** | **200** |
| **Amaranth (Amaranthus caudatus L, semen) dry extract** | **50** | **100** |
| **Hydroxypropyl cellulose** | **25** | **50** |
| **Silicon dioxide** | **22** | **44** |
| **Magnesium salts of fatty acids** | **17** | **34** |
| **Cross-linked sodium carboxymethyl cellulose** | **12** | **24** |
| **Carnauba wax** | **0.1** | **0.2** |
| **Total weight** | **1171.1** | **2342.2** |

### Composition 2

Composition in the form of a tablet or sachet or orosoluble stick or gel stick (1 dosage form once a day) containing:
450 mg diosmin;
50 mg hesperidin;
500 mg pumpkin (dry ext., seeds);
100 mg dry extract of amaranth (dry ext., seeds); and
optionally, pharmaceutically acceptable or food-grade additives and/or excipients.

### Composition 3

Composition in the form of a tablet or sachet or orosoluble stick or gel stick (1 dosage form twice a day) containing:
200 mg diosmin;
22.2 mg hesperidin;
250 mg pumpkin (dry ext., seeds);
50 mg dry extract of amaranth (dry ext., seeds); and
optionally, pharmaceutically acceptable or food-grade additives and/or excipients.

### EXAMPLE 1

The objective of the study is to assess different formulations aimed at countering venous insufficiency after intestinal absorption to avoid possible side effects or irritations following oral administration. The tested mixtures are indicated below. These mixtures were compared with the DFN and FBN commercial products, the formulations of which are also indicated below.

**Table 1**

| FORMULA | COMPONENTS | DOSE |
|---|---|---|
| Formula D | Birch leaf dry extract 20:01 | 200 mg |
| | Hypersmin^{™} (diosmin 400mg; Hesperidin 44.4mg) | 514 mg |
| | Hesperidin | 5.6 mg |
| | Sweet clover dry extract at 20% cont. in Coumarin | 60 mg |
| Formula E | Birch leaf dry extract 20:01 | 200 mg |
| | Hypersmin^{™} (diosmin 450mg; Hesperidin 50mg) | 578 mg |
| | Sweet clover dry extract at 17% cont. in Coumarin | 60 mg |
| MIX 1 (according to the invention) | Hypersmin^{™} (450 mg Diosmin, 50 mg Hesperidin) | 578 mg |
| | Amaranth | 100 mg |
| | Pumpkin | 500 mg |
| MIX 2 | Hypersmin^{™} (400 mg Diosmin, 44.4 mg Hesperidin) | 444.4 mg |
| | hesperidin | 5.6 mg |
| | Amaranth | 100 mg |
| | Pumpkin | 500 mg |

**Table 2**

| COMMERCIAL PRODUCT FORMULATIONS (COMPARATIVE) | | |
|---|---|---|
| FORMULA | COMPONENTS | DOSE |
| FBN | Birch leaf dry extract 20:01 | 200 mg |
| | diosmin | 300 mg |
| | diosmetin | 100 mg |
| | Hesperidin | 50 mg |
| | Sweet clover dry extract at 17% cont. in Coumarin | 60 mg |
| | of which Coumarin | 10.20 mg |
| DFN | diosmin | 450 mg |
| | hesperidin | 50 mg |

### Table 3

### Experimental protocol

**Step 1:** Well-designed *in vitro* models, called Transwell^{®}, were used for the absorption study. They provide for the use of human intestinal epithelial cells, Caco-2 cell line and HT-29MTX cell line, widely used and validated as a human intestinal epithelial cell model for absorption studies on compounds administered orally.

An update of the 3D *in vitro* model approved by the FDA and EMA was used to test absorption and the mechanisms of transport across the intestinal barrier, by performing:
- Analysis of cell viability through the MTT assay
- Analysis of barrier integrity through TEER
- Analysis of permeability through a fluorescent probe

The cells seeded in the Transwell^{®} insert were maintained in a complete medium, changed every other day for 21 days prior to the stimulations, in order to ensure the maturation and formation of intestinal microvilli. Maturation was complete when a transepithelial resistance (TEER) value ≥ 500± 50/cm² was reached.

**Step 2:** Starting from intestinal passage. An *in vitro* model was created that mimics the alteration of venous circulation, to test the performance of both the venous walls and the venous valves following administration of the new formulations. It was planned to recreate a model that recreates the 3D endothelial cell-extracellular matrix (ECM) interaction in a reliable and reproducible manner, according to the method described in Felice F. et al. "Effect of aminaphtone on in vitro vascular permeability and capillary-like maintenance". Phlebology. 2018;33(9):592-599.

This *in vitro* model imitates the key features of the vessel physiology during venous insufficiency following increased destruction of the ECM and venous pressure due to the KCI inducing agent. Such *in vitro* model is indicated below, for brevity, as "venous insufficiency *in vitro* model".

The purpose of this part of the analysis is to examine:
- Analysis of TNFα production
- Activity of the NO vasodilator agent
- Analysis of MMP9, involved in remodelling of the ECM
- Activity of elastin: involved in the processes of maintaining vascular tone

### Analysis

### MTT assay of cell viability

All cell types were subjected to the *in vitro* toxicology assay kit based on MTT (Merck Life Science, Rome, Italy). After stimulation, the cells were incubated in an incubator for two hours at 37°C with 1% MTT dye. Using comparable quantities of MTT solution, the purple formazan crystals were dissolved. Cell viability was assessed by measuring absorbance at 570 nm and correction at 690 nm (Infinite 200 Pro MPlex, Tecan, Mannedorf, Switzerland). The results were compared with the control (untreated samples, represented by the 0% line) and were indicated as the mean (%) SD of five separate tests conducted in triplicate.

### TEER and Papp analyses

The Transwell^{®} technology was used to replicate the intestinal barrier *in vitro* model. After 21 days of culture in a complete medium, before simulations, the transepithelial electrical resistance (TEER) of the CaCo-2 cells was measured using EVOM3 with STX2 stick electrodes (World Precision Instruments, Sarasota, FL, USA). This monitoring allowed maturation of the intestinal epithelium and the emergence of an adequate paracellular mechanism around the 21^{st} day to be assessed. Before stimulation, the culture medium on the apical side was brought to a pH of 6.5 of the lumen of the small intestine. The pH of the basolateral side was brought to 7.4, indicative of blood. In order to exclude degradation of the integrity of the apical side, the TEER values were analysed at each stimulation interval. Once the cells had been treated with all the substances in n intervals from 1-6 hours, they were subjected to Papp analysis (cm/s), which quantifies permeability. The test formula is the following: $Papp = dQ / dt \to ∣ 1 / m 0 \to ∣ 1 / A \to ∣ V Donor$
where: dQ: indicates the quantity of transported material, expressed in nanomoles (nmol) or micrograms (µg);
dt: indicates the duration of the incubation period, expressed in seconds (s);
m0: indicates the initial quantity of substrate applied to the donor compartment, expressed in micrograms (µg) or nanomoles (nmol);
A: area outside the Transwell^{®} membrane, measured in cm²;
VDonor: the volume of liquid (in cm³) in the donor compartment.

### ELISA TNFα kit

Following normal procedure, the TNFα ELISA kit (Merck Life Science, Roma, Italy) was used to measure the quantity of TNFα in the Huvec cells. Using a spectrophotometer (Infinite 200 Pro-MPlex, Tecan, Männedorf, Switzerland), the colorimetric intensity was measured at 450 nm. Creating a calibration curve between 24.58 pg/ml and 6000 pg/ml, the results were calculated and presented as a percentage versus the control of five independent tests performed in triplicate.

### Analysis of nitric oxide production

Following the manufacturer's instructions, a kit (Griess assay, Promega, Italy) was used to measure the production of nitric oxide (NO) following stimulations. A spectrophotometer (Infinite 200 Pro-MPlex, Tecan, Männedorf, Switzerland) was used to measure absorbance of the samples at a wavelength of 520-550 nm. In relation to the standard curve produced with standard nitrate, the results were presented as a percentage (%) normalised versus the untreated samples. Five independent tests were conducted in triplicate and the results were presented as mean ± SD (%) versus the control (line 0).

### Analysis of MMP9

The Human MMP-9 ELISA Kit - Quantikine (R&D Systems, Minneapolis, MN, USA) was used to analyse the levels of MMP9 in the Huvec cells in compliance with the manufacturer's instructions. Absorbance at 450 nm was measured with a spectrometer (Infinite 200 Pro MPlex, Tecan, Männedorf, Switzerland). The standard curve, between 0.3-20 ng/mL, was compared with the data. The results of five different experiments were then presented in triplicate as a percentage (%) versus the control (line 0).

### Elastin ELISA kit

The Human Elastin ELISA kit (Abcam, Cambridge, United Kingdom) was used to measure the elastin in the Huvec cells in accordance with the manufacturer's instructions. A spectrometer (Infinite 200 Pro MPlex, Tecan, Männedorf, Switzerland) was used to quantify absorbance at 450 nm. In relation to the standard curve (0.19 - 12 ng/mL), the results of five separate tests conducted in triplicate were presented as a normalised percentage (%) versus the untreated samples (line 0).

### Statistical analysis

For each experimental technique, the data were presented as mean ± SD of a minimum of 5 biological replicates, performed in triplicate. Using GraphPad Prism 10.2.3 (GraphPad Software, La Jolla, CA, USA), the one-way ANOVA was used with the Mann-Whitney U test or the Bonferroni post hoc test, depending on the circumstances, to perform statistical comparisons between the groups. The threshold for statistical significance is p<0.05. The experimental data were normalised to the control values, fixed at 0%.

### Results

### Step 1

### Analysis of toxicity, of barrier integrity

As shown in Figure 1, MIX 1 and MIX 2 are capable of increasing cell viability versus the control (p<0.05), but also versus FBN and DFN 500mg (p<0.05) for the entire treatment. The TEER analysis confirms the active role of the MIX1 and MIX2 mixtures supporting the absorption thereof (Figure 2).

### Evaluation of permeability and bioavailability

MIX1 and MIX2 have a greater effect than the control (p<0.05), Formula D, Formula E and the FBN and DFN 500 mg commercial products, as shown in table 4 below and in Figure 3. In Table 4, the Papp values in which data < 0.2 × 10⁻⁶ cm/s indicate a very low absorption with a bioavailability < 1%. Data between 0.2 × 10⁻⁶and 2 × 10⁻⁶ cm/s with bioavailability comprised between 1 and 90%. And the data > 2 × 10⁻⁶ cm/s indicate a very good absorption with a bioavailability higher than 90%. The percentage of absorption of the derived substances under examination by means of the Papp values is indicated in Table 4 and Figure 3.

**Table 4**

| | **2H** | **3H** | **4H** | **5H** | **6H** |
|---|---|---|---|---|---|
| MIX 1 | 85.76% | 93.72% | 96.17% | 91.23% | 84.90% |
| MIX 2 | 93.29% | 98.23% | 99.04% | 95.41% | 88.91% |
| Formula D | 43.78% | 50.77% | 67.32% | 57.44% | 53.43% |
| Formula E | 58.11% | 76.05% | 80.26% | 75.32% | 74.66% |
| DFN 500 mg | 35.89% | 45.86% | 60.33% | 55.90% | 52.39% |
| FBN | 39.69% | 41.21% | 69.14% | 60.31% | 52.43% |

### Step 2

Evaluation of the inflammatory state at endothelial level after inducing vascular damage

For evaluation of the inflammatory process, the level of TNFα found following treatment performed on the venous insufficiency *in vitro* model that recreates the 3D endothelial cell-extracellular matrix (ECM) interaction with the mixtures indicated in Tables 2 and 3 was evaluated.

Formula E, MIX 1 and MIX 2 reduce activation of the inflammatory processes to a greater extent than the DFN 500 mg and FBN commercial products (p<0.05).

MIX 1 and MIX 2 show a similar effect. Both the Mixes have a significant effect not only compared to the commercial products, but also compared to Formulas D and E, thus demonstrating the superiority of these two new Mixes in reducing inflammation at endothelial level (Figure 4).

### Evaluation of NO, promoter of vascular permeability

The mixtures indicated on Table 2 were tested on the venous insufficiency *in vitro* model and the production of NO, promoter of vascular permeability, was evaluated. These mixtures were compared with the FBN and DFN commercial products in terms of NO production.

Mixtures D and E increase the production of NO as a vasodilator agent, relative to the endothelial damage in a similar manner to the FBN commercial product, but are not statistically significant relative to DFN 500 mg.

MIX 1 and MIX 2 have a significant effect not only compared to the commercial products, but also compared to mixtures D and E, thus demonstrating the superiority of these two new Mixes in promoting NO production (Figure 5).

### Evaluation of ECM remodelling

Matrix metalloproteinases (MMP) play an important role in maintaining the structure and function of the venous wall. MMPs cause degradation of the extracellular matrix (ECM) proteins, such as collagen and elastin, and could have further effects on the endothelium, on proliferation, on Ca²⁺ signalling and on contraction.

In order to evaluate remodelling of the extracellular matrix (ECM), the level of MMP-9 induced by treatment of the venous insufficiency *in vitro* model with the mixtures indicated on Tables 2 and 3 was evaluated.

As can be observed in Figure 6, mixture D reduces activation of the MMP-9 in an equivalent manner to FBN. Whereas mixture E does so in a superior manner to both the commercial products, although not significantly. In this case as well, MIX 1 and MIX 2 act in a similar manner on the endothelium. Both the MIXES have a significant effect not only compared to the commercial products, but also compared to mixture D and E, thus demonstrating the superiority of these two new Mixes in modulating the action of the MMPs.

### Evaluation of maintaining of vascular tone

In order to evaluate maintaining of vascular tone, the level of elastin induced by treatment of the venous insufficiency *in vitro* model with the mixtures of Tables 2 and 3 was evaluated.

It was observed that the Mixes have a superior effect compared to mixtures D and E and compared to the FBN and DFN commercial products, with significant results (Figure 7).

In conclusion, the Applicant has observed that MIXES 1 and 2 are better than the other mixtures tested and than the DFN and FBN commercial products.

### EXAMPLE 2

In example 2, the mixtures indicated on Table 5 were tested and compared with the FBN and DFN 500 mg commercial products, the formulations of which are indicated on Table 2.

**Table 5**

| FORMULA | COMPONENTS | DOSE |
|---|---|---|
| MIX 1 | Hypersmin^{™} (Diosmin 450 mg; Hesperidin 50 mg) | 578 mg |
| | Amaranth | 100 mg |
| | Pumpkin | 500 mg |
| Formula F | Birch leaf dry extract 20:01 | 200 mg |
| | Hypersmin^{™} (Diosmin 450 mg; Hesperidin 50 mg) | 578 mg |
| | Sweet clover dry extract at 20% cont. in Coumarin | 60 mg |
| | Amaranth | 50 mg |
| | Pumpkin | 40 mg |

### Step 1

In step 1 of this experiment, cell viability was evaluated through an MTT assay, barrier integrity through TEER and permeability through a fluorescent probe.

Well-designed *in vitro* models, called Transwell^{®}, were used for the absorption study. They provide for the use of human intestinal epithelial cells, Caco-2 cell line and HT-29MTX cell line, widely used and validated as a human intestinal epithelial cell model for absorption studies on compounds administered orally. The cells seeded in the Transwell^{®} insert were maintained in a complete medium, changed every other day for 21 days prior to the stimulations, in order to ensure the maturation and formation of intestinal microvilli. Maturation was complete when a transepithelial resistance (TEER) value ≥ 500± 50/cm² was reached. It was observed that MIX 1 and Formula F are capable of increasing cell viability versus the control (p<0.05), but also versus FBN and DFN 500mg (p<0.05) for the entire treatment. MIX 1 induces greater beneficial effects than Formula F, both insofar as concerns cell viability and TEER analysis (Figure 8).

### Step 2

In step 2, MIX 1 and Formula F were tested on the venous insufficiency *in vitro* model described in example 1.

In this step, the following analyses were performed:
- Analysis of TNFα production
- Analysis of the activity of elastin: involved in the processes of maintaining vascular tone
- Analysis of the activity of the NO vasodilator agent
- Analysis of MMP9, involved in remodelling of the ECM

The Applicant observed that the mixture according to the invention performs better than formula F and the FBN and DFN 500 mg commercial products, as shown in Figures 9 to 11.

Furthermore, it was observed that the mixture according to the invention MIX 1 increases the production of NO as the vasodilator agent, relative to the endothelial damage, to a greater extent than formula F (not shown).

### Example 2 conclusions

In conclusion, the Applicant has observed that MIX 1 is also better than Formula F and than the DFN and FBN commercial products.

### EXAMPLE 3

At this point, the Applicant evaluated two different dosages/posologies of the same product to counter venous insufficiency after intestinal absorption.

In detail, the two dosages tested are:
Hypersmin 578mg (450mg Diosmin, 50mg Hesperidin), Pumpkin seeds dry ext. 325 mg, Amaranth 65 mg (Formula New)
Hypersmin 289mg (225mg Diosmin, 25mg Hesperidin), Pumpkin seeds dry ext. 250 mg, Amaranth 50 mg (Formula New 2.0).

These dosages were compared with the DFN commercial product (tables 6 and 7).

**Table 6: Protocol A: 1 tablet/per day (indicated in Figures 12- 15 as "tab/per day"**

| FORMULA | COMPONENTS | DOSE |
|---|---|---|
| DFN | Diosmin | 450 mg |
| | Hesperidin | 50 mg |
| Formula New | Hypersmin^{™} (450 mg Diosmin; 50 mg Hesperidin) | 578 mg |
| | Amaranth | 65 mg |
| | Pumpkin | 325 mg |

**Table 7: Protocol B: 2 tablets/per day (indicated in Figures 12-15 as "tab/per day")**

| FORMULA | COMPONENTS | DOSE |
|---|---|---|
| DFN | Diosmin | 450 mg |
| | Hesperidin | 50 mg |
| Formula New 2.0 | Hypersmin^{™} (225 mg Diosmin; 25 mg Hesperidin) | 289 mg |
| | Amaranth | 50 mg |
| | Pumpkin | 250 mg |

In both protocols (A and B), the two new formulas induce a reduction of degradation of the ECM (to be intended as % of the level of MMP9 vs. control) compared to DFN (1 tablet/per day and 2 tablets/per day). Formula New 2.0 remains significant on DFN 2 capsules during all the timings analysed, with the exception of 12h.

In both protocols (A and B), the two new formulas induce:
- a reduction in production of TNFα at endothelium level, after inducing vascular damage, greater than DFN (1 tablet/per day and 2 tablets/per day), (Figure 12).
- an increase in maintaining of vascular permeability greater than DFN (1 tablet/per day and 2 tablets/per day), measuring NO production;
- a reduction in ECM degradation compared to DFN (1 tablet/per day and 2 tablets/per day), measuring the level of MMP-9; and
- an increase in maintaining of vascular tone greater than DFN (1 tablet/per day and 2 tablets/per day).

In all the parameters tested, Formula New and Formula New 2.0 maintain a higher efficacy than DFN (1 or 2 tablets/per day).

## Claims

1. A mixture comprising or, alternatively, consisting of:
i) diosmin;
ii) hesperidin;
iii) a pumpkin extract; and
iv) an amaranth extract, where said diosmin and said hesperidin are present in a diosmin:hesperidin weight ratio comprised from 15:1 to 8:1.

2. The mixture according to claim 1, where the diosmin is present in a quantity by weight comprised between 20% and 80% and where the hesperidin is present in a quantity by weight comprised between 1% and 25% relative to the total weight of the mixture.

3. The mixture according to claim 1 or 2, where the diosmin is present in a quantity by weight comprised between 30% and 60% and where the hesperidin is present in a quantity by weight comprised between 2% and 20% relative to the total weight of the mixture.

4. The mixture according to any one of claims 1 to 3, where the pumpkin extract is obtained from the seeds and/or from the pulp of a plant belonging to the *Cucurbita maxima* species.

5. The mixture according to any one of claims 1 to 4, where the amaranth extract is obtained from the seeds of a plant belonging to the *Amaranthus caudatus* and/or *Amaranthus cruentus* and/or *Amaranthus hypochondriacus* species.

6. The mixture according to claim 5, where the amaranth extract is obtained from the seeds of a plant belonging to the *Amaranthus caudatus* species.

7. The mixture according to any one of claims 1 to 6, wherein said mixture comprises:
the pumpkin extract in a quantity by weight, relative to the total weight of the mixture, comprised from 20% to 80%, preferably from 30% to 60%, more preferably from 40% to 50%; and
the amaranth extract in a quantity by weight, relative to the total weight of
the mixture, comprised from 1% to 20%, preferably from 5% to 15%, more preferably from 9% to 12%.

8. A composition comprising a mixture according to any one of claims 1 to 7 and, optionally, pharmaceutically acceptable or food-grade additives and/or excipients.

9. The composition according to claim 8, formulated for oral administration, preferably as tablets, more preferably, gastro-resistant tablets, extended-release tablets, sublingual tablets, orodispersible tablets, capsules, more preferably hard or soft capsules, coated tablets, oral powders, oral granules, single-dose sachets, oral suspensions, oral solutions, syrups, elixirs, oral drops, oral emulsions, oral pastes, sticks, orosoluble sticks or gels.

10. The mixture according to any one of claims 1 to 7 or the composition according to claims 8 or 9, for use as a medicament.

11. The mixture according to any one of claims 1 to 7 or the composition according to claims 8 or 9, for use in the treatment or in the prevention of a venous disorder, preferably a peripheral venous disorder or a pathology caused by or associated with said venous disorder.

12. The mixture for the use according to claim 11, where the venous disorder is chosen from among chronic venous insufficiency, oedema of lower limbs, telangiectasia, varicose veins, reticular veins, capillary fragility, haemorrhoids, skin hyperpigmentation (ochre dermatitis), lipodermatosclerosis, varicose eczema, venous ulcers, deep vein thrombosis (DVT), superficial thrombophlebitis, tissue fibrosis, stasis skin infections, functional venous pain, orthostatic oedema, sensation of warmth or burning along the vein path.

13. The mixture for the use according to claim 11 or 12, through an increase in the venous wall tone, improvement of the contractility and elasticity of the venous wall, reduction of tissue inflammation, decrease in vascular permeability, improvement of lymphatic drainage, protection of capillaries.

14. A non-therapeutic use of the mixture according to any one of claims 1 to 7 or the composition according to claim 8 or 9 for maintaining or improving microcirculation in an individual.

15. A dietary supplement comprising the mixture according to any one of claims 1 to 7 or the composition according to claim 8 or 9.
